# EUROPÄISCHE PATENTSCHRIFT

(11) **EP 0 995 412 B1**
(45) Veröffentlichungstag und Bekanntmachung des Hinweises auf die Patenterteilung: **31.03.2004**
(21) Anmeldenummer: 99810861.7
(22) Anmeldetag: 24.09.1999
(51) Int. Cl.: A61F 2/46, A61F 2/34

(54) **Künstliche Gelenkpfanne**
Artificial joint cup
Cupule articulaire artificielle

(30) Priorität: 23.10.1998 EP 98811065
(43) Veröffentlichungstag der Anmeldung: 26.04.2000
(73) Patentinhaber: Centerpulse Orthopedics Ltd., 6340 Baar (CH)
(72) Erfinder: Wymann, Burckhard, 8353 Elgg (CH)
(74) Vertreter: Manitz, Finsterwald & Partner Gbr

(56) Entgegenhaltungen:
- EP-A- 0 826 347
- WO-A-95/01139
- WO-A-98/55051
- DE-A- 19 611 250
- DE-U- 29 516 473
- FR-A- 2 398 490
- US-A- 5 641 323

## Beschreibung

Die Erfindung betrifft eine künstliche Gelenkpfanne gemäss Anspruch 1.

Künstliche Gelenkpfannen im allgemeinen, insbesondere aber Hüftgelenkpfannen, die in das *acetabulum* eines Patienten implantiert werden, umfassen in der Regel folgende wesentliche Teile: Eine Schale einerseits, die bei zementfreier Implantation meistens aus Titan bzw. einer Titanlegierung hergestellt ist (bei zementierter Implantation z.B. aus einer Kobalt-Chrom-Legierung), und einen in diese Schale einsetzbaren Einsatz andererseits, der in der Regel entweder aus Polyethylen oder Keramik hergestellt ist oder metallisch ist. Der Einsatz weist eine Gelenkfläche auf, auf welcher die Gelenkkugel, bei einer Hüftgelenkpfanne die femurseitige Gelenkkugel, beweglich ist. Beim Implantieren einer solchen künstlichen Hüftgelenkpfanne wird typischerweise zunächst die Schale im *acetabulum* befestigt - zahlreiche Arten der Befestigung sind hierzu bekannt. Nach dem Befestigen der Schale wird der Einsatz in die Schale eingebracht, und zwar derart, dass er nach dem Einbringen fest in der Schale angeordnet ist.

Das feste Anordnen in der Schale kann auf verschiedene Art und Weise erfolgen: Bei Einsätzen aus Polyethylen kann dies beispielsweise dadurch erfolgen, dass der Einsatz (auch als "Inlay" bezeichnet) durch eine Schnappverbindung in der Schale einschnappt, dass also zwei sich hinterschneidende Partien aneinander vorbei gleiten und auf diese Weise eine Schnappverbindung eingehen, die nicht ohne besondere Massnahmen gelöst werden kann.

Bei härteren Materialien der Einsätze wie Keramik oder Metall werden seit einiger Zeit Konusverbindungen eingesetzt, bei denen die feste Verbindung mit Hilfe einer konischen Fläche auf der Aussenwand des Einsatzes und einer entsprechenden konischen Fläche auf der Innenwand der Schale zustande kommt. Der Einsatz aus Keramik bzw. aus Metall wird in einem Klemmsitz in der Schale festgehalten, der nicht ohne besondere Massnahmen gelöst werden kann.

Auch wenn solche künstlichen Gelenkpfanne sehr beständig sind und auch hohe Belastungen vertragen können, kann es doch aus verschiedenen Gründen nach einiger Zeit erforderlich werden, eine solche künstliche Pfanne einer Revision zu unterziehen. In diesem Falle möchte man natürlich nicht die gesamte Gelenkpfanne erneuern, sondern lediglich den Einsatz, weil die Schale in der Regel fest und gut mit dem Knochenmaterial des *acetabulums* verbunden ist. Andererseits ist aber der Einsatz fest mit der Schale verbunden und kann nicht so ohne weiteres von der Schale gelöst werden.

Bei Einsätzen aus Polyethylen (Schnappverbindung zwischen Einsatz und Schale) erfolgt das Lösen des Einsatzes von der Schale - im oben erläuterten Beispiel also das Lösen der Schnappverbindung - beispielsweise derart, dass ein Hilfswerkzeug nach dem Prinzip eines Korkenziehers durch den Einsatz aus Polyethylen hindurch geschraubt wird. Das vordere Ende des Hilfswerkzeugs stützt sich beim weiteren Einschrauben des Hilfswerkzeugs auf der Schale (Titan) ab und zwingt den Einsatz aus der Schale heraus, indem die sich hinterschneidenden Partien von Einsatz und Schale (gegen die Rückhaltekraft der Schnappverbindung) wieder aneinander vorbeigleiten, dieses Mal aber in umgekehrter Richtung wie beim Einbringen des Einsatzes, wodurch die Schnappverdinung gelöst wird.

Bei Einsätzen aus härteren Materialien wie Keramik oder Metall, die über einen Konusklemmsitz fest in der Schale angeordnet sind, müssen andere Methoden angewandt werden. Eine bekannte Methode zum Lösen eines solchen Konusklemmsitzes besteht darin, dass man auf die Schale - jedoch nicht auf den Einsatz - einen Schlagimpuls überträgt (zum Beispiel mit einem geeigneten Hilfswerkzeug), wodurch der Konusklemmsitz gelöst wird.

Es ist unmittelbar einleuchtend, dass beide Methoden nicht gerade komfortabel für den Chirurgen bzw. Orthopäden sind, der die Operation durchführt. Das Einschrauben des Hilfswerkzeugs bei Polyethyleneinsäzten ist vergleichsweise mühsam. Das Lösen von Keramik- oder Metalleinsätzen mittels Schlagimpuls ist ebenfalls einigermassen mühsam, weil ein entsprechendes Hilfswerkzeug zum Ubertragen des Schlagimpulses zunächst an der Pfanne angesetzt und gegen Abgleiten gesichert werden muss und erst dann der Schlagimpuls erzeugt werden kann. Darüberhinaus ist es bei dieser Vorgehensweise auch so, dass die Übertragung des Schlagimpulses sich negativ auf das Knochenmaterial auswirken kann. Darüberhinaus ist es auch nicht gesichert, ob durch Übertragung eines Schlagimpulses auf die Schale Einsatze, die über mehrere Jahre hinweg im Körper des Patienten in der Schale eingesetzt waren, zuverlässig gelöst werden.

In der DE-A 196 11250 ist eine Gelenkprothese mit einem Trägerteil und einem Einsatz beschrieben, bei der der Einsatz mit Hilfe eines Aushebewerkzeugs mechanisch aus dem Trägerteil herausgedrückt wird.

Es ist eine Aufgabe der Erfindung, eine künstliche Gelenkpfanne vorzuschlagen, die eine einfache und gleichzeitig schonende und sichere Methode zum Lösen des Einsatzes von der Schale zulässt, wobei die Methode sowohl zum Lösen von Einsätzen aus Polyethylen als auch zum Lösen von Einsätzen aus härteren Materialien wie Keramik oder Kunststoff geeignet ist.

Diese Aufgabe wird gelöst durch die Merkmale des Anspruchs 1. Zum Lösen des Einsatzes von der Schale wird ein Fluid zwischen den Einsatz und die Schale eingebracht, welches das Lösen des Einsatzes von der Schale bewirkt. Dies ist vergleichsweise einfach, wie noch genauer erläutert werden wird, und erfolgt auch ohne Belastung für das umliegende Knochengewebe, in welchem die Schale verankert ist. Nach dem Lösen des Einsatzes kann der Einsatz einfach aus der Schale entnommen und ein neuer Einsatz in die Schale eingebracht werden.

Vorzugsweise wird als Fluid eine Flüssigkeit, insbesondere eine körperverträgliche Flüssigkeit, verwendet. Dies kann beispielsweise eine körperverträgliche Kochsalzlösung sein. Grundsätzlich ist es auch möglich, als Fluid ein Gas zu verwenden, wegen der Inkompressibiliät von Flüssigkeiten wird aber in der Mehrzahl der Fälle einer Flüssigkeit der Vorzug einzuräumen zu sein.

Speziell bei Einsätzen aus härteren Materialien wie Keramik oder Metall ist es regelmässig so, dass bei fest in die Schale eingebrachtem Einsatz zwischen dem Boden der Schale und dem Boden des Einsatzes noch ein (Zwischen-) Raum vorhanden ist. Demzufolge wird bei einem Ausführungsbeispiel das Fluid bzw. die Flüssigkeit in diesen Raum zwischen dem Boden der Schale und dem Boden des Einsatzes hinein eingebracht, bis sich der Einsatz von der Pfanne löst, also der Konusklemmsitz gelöst ist. Nach dem Lösen des Einsatzes von der Schale kann dann der Einsatz einfach aus der Schale entnommen und ein neuer Einsatz eingebracht werden.

Bei Einsätzen aus Polyethylen besteht die Verbindung von Einsatz und Schale häufig in einem Schnappverschluss nahe dem Boden des Einsatzes. Der Boden des Einsatzes aus Polyethylen steht mehr oder weniger auf dem Boden der Schale auf, weshalb hier - anders als bei Einsätzen aus härteren Materialien wie Keramik oder Metall - praktisch kein Raum zwischen dem Boden der Schale und dem Boden des Einsatzes vorhanden ist, in welchen das Fluid oder die Flüssigkeit eingebracht werden kann. Aus diesem Grund kann in der Schale eine nach oben hin offene Nut, z. B. eine Ringnut, vorgesehen sein, in welche das Fluid bzw. die Flüssigkeit eingebracht wird. Durch das in diese Nut (unter Druck) eingebrachte Fluid bzw. die Flüssigkeit kann der Einsatz aus der Schale herausgedrückt werden, bis der Schnappverschluss gelöst ist. Nach dem Lösen des Schnappverschlusses lässt sich der Einsatz auf einfache Weise aus der Schale entfernen.

Im allgemeinen lässt sich also sagen, dass bei dieser Variante das Fluid bzw. die Flüssigkeit in einen unterhalb des Bodens des Einsatzes sich erstreckenden Raum hinein eingebracht wird. Dies kann - wie oben beschrieben - entweder ein Raum sein, der sich zwischen dem Boden der Schale und dem Boden des Einsatzes erstreckt (z.B. Konusklemmsitz bei Einsätzen aus härteren Materialien) oder eine in der Schale vorgesehene nach oben offene Nut, z.B. die genannte Ringnut (z.B. bei am Boden der Schale aufstehenden Einsätzen aus Polyethylen).

Das Fluid bzw. die Flüssigkeit kann auch in einen Raum zwischen der Seitenwand der Schale und der Seitenwand des Einsatzes hinein eingebracht werden, was insbesondere bei Einsätzen aus härteren Materialien wie Keramik oder Metall interessant ist. Diese Massnahme kann entweder alleine oder zusätzlich zum Einbringen des Fluids in den Raum zwischen dem Boden der Schale und dem Boden des Einsatzes erfolgen.

Weitere vorteilhafte Ausgestaltungen ergeben sich aus der nachfolgenden Beschreibung von Ausführungsbeispielen der erfindungsgemässen künstlichen Gelenkpfanne. Es zeigen in schematischer Darstellung im Schnitt:
- Fig. 1: einen Ausschnitt aus einer künstlichen Hüftgelenkpfanne mit einem Einsatz aus härterem Material wie Keramik oder Metall, der mittels einer Konusverbindung fest in der Schale angeordnet ist.
und
- Fig. 2: einen Ausschnitt aus einer künstlichen Hüftgelenkpfanne mit einem Einsatz aus Polyethylen, der mittels einer Schnappverbindung fest in der Schale angeordnet ist.

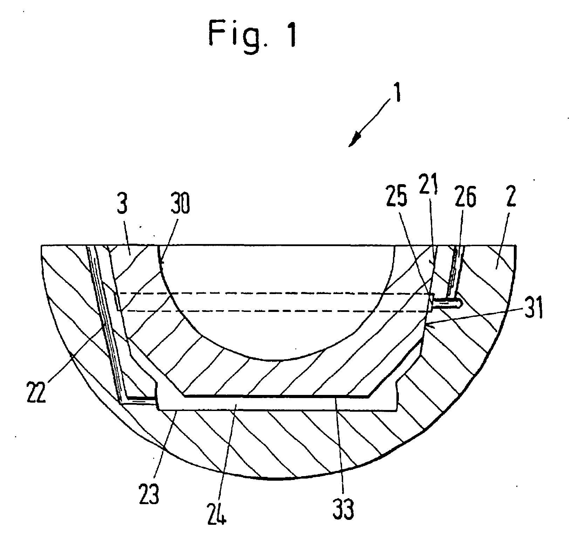

In Fig. 1 erkennt man eine künstliche Gelenkpfanne, hier eine künstliche Hüftgelenkpfanne 1, welche eine Schale 2 und einen Einsatz 3 umfasst. Der Einsatz 3 ist in diesem Ausführungsbeispiel aus einem härteren Material hergestellt, beispielsweise aus Keramik oder Metall. Er weist eine Gelenkfläche 30 auf, auf welcher die femurseitige Gelenkkugel (nicht dargestellt) beweglich ist. Typischerweise stellt diese Gelenkfläche 30 einen Ausschnitt aus einer Kugelfläche dar.

Der Einsatz 3 ist in der Schale 2 mit Hilfe einer Konusverbindung fest angeordnet. Diese Konusverbindung kommt mit Hilfe einer konischen Fläche der Seitenwand 31 (Aussenwand) des Einsatzes 3 und einer entsprechenden konischen Fläche der Seitenwand 21 (Innenwand) des Einsatzes 2 zustande. Diese bilden beim Einbringen des Einsatzes 3 in die Schale 2 einen Klemmsitz, sodass der Einsatz 3 bei einer Revision nicht ohne besondere Massnahmen von der Schale 2 gelöst werden kann.

Um den Einsatz 3 nun auf einfache und vor allen Dinge auch für das umliegende Knochenmaterial schonende Art und Weise von der Schale 2 lösen zu können, ist hier in der Schale 2 ein Kanal 22 (gestrichelt dargestellt) vorgesehen, der sich von der Stirnfläche der Schale 2 beginnend bis zu einem Bereich nahe dem Boden 23 der Schale 2 erstreckt. Dort mündet der Kanal 22 in einen Raum 24 hinein, der sich zwischen dem Boden 23 der Schale 2 und dem Boden 33 des Einsatzes 3 erstreckt.

In diesen Raum 24 hinein wird nun das Fluid, insbesondere eine körperverträgliche Flüssigkeit wie beispielsweise eine körperverträgliche Kochsalzlösung, so lange eingebracht, bis der Klemmsitz der Konusverbindung zwischen der Seitenwand 31 des Einsatzes 3 und der Seitenwand 21 der Schale 2 gelöst ist. Hierzu ist in der Regel kein unpraktikabler Druck erforderlich, der Druck beträgt allenfalls bis hin zu einigen bar.

Zusätzlich kann auch noch Fluid in einen Raum zwischen der Seitenwand 31 des Einsatzes 3 und der Seitenwand 21 der Schale 2 eingebracht werden. Dieser Raum wird beispielsweise durch eine in der Seitenwand 21 der Schale 2 vorgesehenen umlaufenden Nut 25 gebildet. Durch einen Kanal 26 gelangt das Fluid, beispielsweise die genannte körperverträgliche Flüssigkeit, in die Nut 25 hinein, füllt zunächst diese Nut 25 und trägt anschliessend dazu bei, dass der Konusklemmsitz des Einsatzes 3 in der Schale 2 gelöst wird.

Die Massnahme, das Fluid in eine umlaufende Nut 25, also in einen Raum zwischen der Seitenwand 31 des Einsatzes 3 und die Seitenwand 21 der Schale 2 hinein einzubringen, ist grundsätzlich unabhängig von der Massnahme, das Fluid zwischen den Boden 23 der Schale 2 und den Boden 33 des Einsatzes 3 einzubringen. Beide Massnahmen können sowohl einzeln als auch zusammen erfolgen.

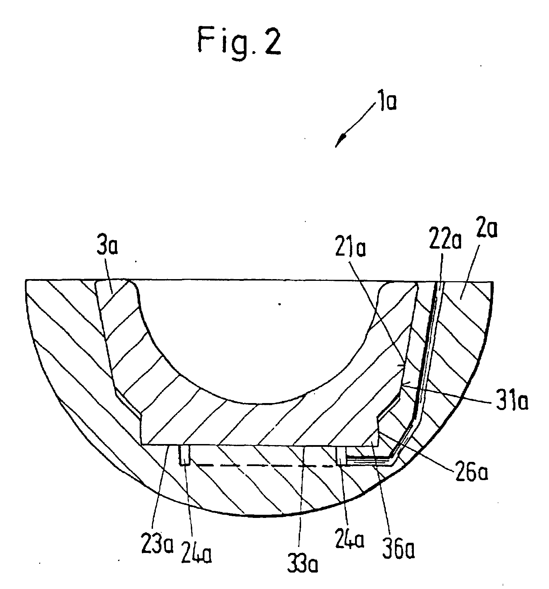

Fig. 2 zeigt ein Ausführungsbeispiel einer künstlichen Gelenkpfanne, erneut einer Hüftgelenkpfanne 1a, bei welcher der Einsatz 3a in der Schale 2a mit Hilfe eines Schnappverschlusses fest angeordnet ist. Der Schnappverschluss wird durch sich hinterschneidende Partien 36a am Einsatz 3a und 26a an der Schale 2a gebildet. Beim Einbringen der Schale 2a gleiten die sich hinterschneidenden Partien 36a und 26a aneinander vorbei, wodurch der Schnappverschluss "einschnappt" und der Einsatz 3a fest in der Schale 2a angeordnet ist. Als Sicherung gegen Verdrehen des Einsatzes 3a gegenüber der Schale 2a können gesonderte Mittel vorgesehen sein (nicht dargestellt).

Zum Lösen des Einsatzes 3a von der Schale 2a muss der Schnappverschluss gelöst werden. also die Partien 36a des Einsatzes 3a und 26a der Schale 2a wieder aneinander vorbeigleiten, allerdings in umgekehrter Richtung wie beim Einbringen des Einsatzes 3a. Hierzu kann ein Kanal 22a vorgesehen sein, der in eine nach oben offene Ringnut 24a hinein mündet, welche in der Schale 2a vorgesehen ist und sich unterhalb des Bodens 33a des Einsatzes 3a erstreckt. Bei diesem Ausfühungsbeispiel der künstlichen Gelenkpfanne 1a erstreckt sich der Einsatz 3a nämlich im wesentlichen bis zum Boden 23a der Schale 2a (anders als bei dem zuvor beschriebenen Ausführungsbeispiel) weil ja die Partie 36a des Einsatzes 3a über die Partie 26a der Schale 2a gleiten muss, um das Einschnappen des Schnappverschlusses zu bewirken Durch Zuführen eines Fluids, vorzugsweise einer körperverträglichen Flüssigkeit wie z.B. einer körperverträglichen Kochsalzlösung, durch den Kanal 22a in die Ringnut 24a hinein kann der Schnappverschluss "geöffnet" werden, indem die Partie 36a des Einsatzes 3a zum Gleiten über die Partie 26a der Schale 2a gezwungen wird. Hierzu ist kein unpraktikabler Druck erforderlich, der Druck beträgt allenfalls bis zu einigen bar.

Gleichwohl ist es prinzipiell auch möglich, den Schnappverschluss nicht in der Nähe des Bodens der Schale vorzusehen, sondern weiter vom Boden entfernt. Dann ist es auch möglich, dass das Fluid - ähnlich wie beim Ausführungsbeispiel gemäss Fig. 1 - in einen Raum zwischen dem Boden des Einsatzes und dem Boden der Schale einzubringen, weil dann der Boden des Einsatzes nicht im wesentlichen bis zum Boden der Schale reichen muss. Auch kann natürlich - ähnlich wie beim Ausführungsbeispiel gemäss Fig. 1 - eine Nut in der Seitenwand der Schale vorgesehen sein, in welche Flüssigkeit eingebracht werden kann, um das Herauslösen des Einsatzes noch zu erleichtern. Als einzige Massnahme zum Lösen des Einsatzes aus der Schale ist in diesem Falle eine solche in der Seitenwand angeordnete Nut jedoch nicht geeignet.

## Patentansprüche

1. Künstliche Gelenkpfanne (1, 1a), welche eine Schale (2, 2a) und einen Einsatz (3, 3a) umfasst, wobei
der Einsatz (3, 3a) fest in der Schale (2, 2a) angeordnet ist,
die Schale (2, 2a) und der Einsatz (3, 3a) so ausgebildet sind, dass bei in die Schale (2, 2a) eingesetztem Einsatz (3, 3a) wenigstens ein Raum (24, 25, 24a) zwischen dem Einsatz (3, 3a) und der Schale (2, 2a) gebildet ist, und
wenigstens ein Kanal (22, 26, 22a) zum Einbringen eines Fluids in den Raum (24, 25, 24a) vorgesehen ist, der sich von der Stirnfläche der Schale beginnend zu dem Raum (24, 25, 24a) erstreckt und in diesen hinein mündet,
wobei der Raum (24, 25, 24a) mit Ausnahme der Mündung des Kanals (22, 26, 22a) bezüglich des verwendeten Fluids gegenüber der Umgebung fluiddicht derart abgeschlossen ist, dass mittels des über den Kanal (22, 26, 22a) in den Raum (24, 25, 24a) einbringbaren Fluids in dem Raum (24, 25, 24a) ein Druck zum Lösen des Einsatzes (3, 3a) von der Schale (2, 2a) aufbaubar ist, und
wobei der Kanal (22, 26, 22a) in der Schale (2, 2a) ausgebildet ist und die Wände des Kanals (22, 26, 22a) durch die Schale (2, 2a) gebildet sind.

2. Künstliche Gelenkpfanne nach Anspruch 1,
**dadurch gekennzeichnet,**
**dass** der Raum (24, 24a) sich unterhalb des Bodens (33, 33a) des Einsatzes (3, 3a) erstreckt.

3. Künstliche Gelenkpfanne nach Anspruch 1 oder 2,
**dadurch gekennzeichnet,**
**dass** der Raum sich zwischen dem Boden (23) der Schale (2) und dem Boden (33) des Einsatzes (3) erstreckt.

4. Künstliche Gelenkpfanne nach einem der Ansprüche 1 bis 3,
**dadurch gekennzeichnet,**
**dass** der Raum (24a) als eine in der Schale (2a) vorgesehene, nach oben durch den Boden (33a) des Einsatzes (3a) geschlossene Ringnut ausgebildet ist.

5. Künstliche Gelenkpfanne nach einem der Ansprüche 1 bis 4
**dadurch gekennzeichnet,**
**dass** der Raum (25) zwischen der Seitenwand (21) der Schale (2) und der Seitenwand (31) des Einsatzes (3) ausgebildet ist.

6. Künstliche Gelenkpfanne nach Anspruch 5,
**dadurch gekennzeichnet,**
**dass** der Raum (25) als umlaufende Nut in der Seitenwand (21) der Schale (2) ausgebildet ist.

7. Künstliche Gelenkpfanne nach einem der Ansprüche 1 bis 6,
**dadurch gekennzeichnet,**
**dass** zwischen Einsatz (3, 3a) und Schale (2, 2a) ein Klemmsitz ausgebildet ist

8. Künstliche Gelenkpfanne nach einem der Ansprüche 1 bis 7,
**dadurch gekennzeichnet,**
**dass** der Einsatz (3, 3a) mittels einer Konusverbindung fest in der Schale (2, 2a) angeordnet ist.

9. Künstliche Gelenkpfanne nach Anspruch 8,
**dadurch gekennzeichnet,**
**dass** zur Ausbildung der Konusverbindung die Seitenwand (21) der Schale (2) und die Seitenwand (31) des Einsatzes (3) mit einer konischen Fläche versehen sind.

10. Künstliche Gelenkpfanne nach einem der Ansprüche 1 bis 9,
**dadurch gekennzeichnet,**
**dass** der Einsatz (3a) mittels einer Schnappverbindung fest in der Schale (2a) angeordnet ist.

11. Künstliche Gelenkpfanne nach Anspruch 10,
**dadurch gekennzeichnet,**
**dass** die Schnappverbindung in der Nähe des Bodens der Schale (2a) angeordnet ist.

12. Künstliche Gelenkpfanne nach Anspruch 10 oder 11,
**dadurch gekennzeichnet,**
**dass** die Schnappverbindung durch sich hinterschneidende Partien (36a, 26a) am Einsatz (3a) und an der Schale (2a) gebildet ist.

13. Künstliche Gelenkpfanne nach einem der Ansprüche 1 bis 12,
**dadurch gekennzeichnet,**
**dass** der Einsatz (3) aus Keramik oder Metall hergestellt ist.

14. Künstliche Gelenkpfanne nach einem der Ansprüche 1 bis 12,
**dadurch gekennzeichnet,**
**dass** der Einsatz (3a) aus Polyethylen hergestellt ist.

## Claims

1. An artificial joint socket (1, 1a) which includes a shell (2, 2a) and an insert (3, 3a), wherein
the insert (3, 3a) is fixedly arranged in the shell (2, 2a);
the shell (2, 2a) and the insert (3, 3a) are made such that, when the insert (3, 3a) is inserted into the shell (2, 2a), at least one space (24, 25, 24a) is formed between the insert (3, 3a) and the shell (2, 2a); and
at least one passage (22, 26, 22a) is provided for the introduction of a fluid into the space (24, 25, 24a), said passage extending, starting from the end face of the shell, to the space (24, 25, 24a) and opening into it;
wherein the space (24, 25, 24a) is closed in a fluid tight manner in relation to the environment with respect to the fluid used, with the exception of the opening of the passage (22, 26, 22a), such that a pressure can be built up in the space (24, 25, 24a) by means of the fluid which can be introduced into the space (24, 25, 24a) via the passage (22, 26, 22a) for the release of the insert (3, 3a) from the shell (2, 2a); and
wherein the passage (22, 26, 22a) is formed in the shell (2, 2a) and the walls of the passage (2, 26, 22a) are formed by the shell (2, 2a).

2. An artificial joint socket in accordance with claim 1, **characterised in that** the space (24, 24a) extends beneath the base (33, 33a) of the insert (3, 3a).

3. An artificial joint socket in accordance with claim 1 or claim 2, **characterised in that** the space extends between the base (23) of the shell (2) and the base (33) of the insert (3).

4. An artificial joint socket in accordance with any of claims 1 to 3, **characterised in that** the space (24a) is formed as a closed ring groove provided in the shell (2a) and upwardly open through the base (33a) of the insert (3a).

5. An artificial joint socket in accordance with any of claims 1 to 4, **characterised in that** the space (25) is formed between the side wall (21) of the shell (2) and the side wall (31) of the insert.

6. An artificial joint socket in accordance with claim 5, **characterised in that** the space (25) is formed as a peripheral groove in the side wall (21) of the shell (2).

7. An artificial joint socket in accordance with any of claims 1 to 6, **characterised in that** a clamping seat is formed between the insert (3, 3a) and the shell (2, 2a).

8. An artificial joint socket in accordance with any of claims 1 to 7, **characterised in that** the insert (3, 3a) is fixedly arranged in the shell (2, 2a) by means of a conical connection.

9. An artificial joint socket in accordance with claim 8, **characterised in that** the side wall (21) of the shell (2) and the side wall (31) of the insert (3) are provided with a conical surface for the formation of the conical connection.

10. An artificial joint socket in accordance with any of claims 1 to 9, **characterised in that** the insert (3a) is fixedly arranged in the shell (2a) by means of a snap-in connection.

11. An artificial joint socket in accordance with claim 10, **characterised in that** the snap-in connection is arranged close to the base of the shell (2a).

12. An artificial joint socket in accordance with claim 10 or claim 11, **characterised in that** the snap-in connection is formed by areas (36a, 26a) on the insert (3a) and on the shell (2a) which undercut one another.

13. An artificial joint socket in accordance with any of claims 1 to 12, **characterised in that** the insert (3) is made of a ceramic material or of metal.

14. An artificial joint socket in accordance with any of claims 1 to 12, **characterised in that** the insert (3a) is made of polyethylene.

## Revendications

1. Glène artificielle (1, 1a), qui comporte une coque (2, 2a) et un élément rapporté (3, 3a), dans laquelle
l'élément rapporté (3, 3a) est solidement agencé dans la coque (2, 2a),
la coque (2, 2a) et l'élément rapporté (3, 3a) sont réalisés de telle sorte que, lorsque l'élément rapporté (3, 3a) est placé dans la coque (2, 2a), au moins un espace (24, 25, 24a) soit formé entre l'élément rapporté (3, 3a) et la coque (2, 2a), et
dans laquelle il est prévu au moins un canal (22, 26, 22a) pour l'introduction d'un fluide dans l'espace (24, 25, 24a), lequel canal s'étendant à partir de la face extérieure de la coque jusqu'à l'espace (24, 25, 24a) et débouche dans celui-ci,
dans laquelle l'espace (24, 25, 24a) est, exception faite de l'ouverture du canal (22, 26, 22a), rendu étanche au fluide utilisé par rapport aux parties situées autour, de telle sorte que, à l'aide du fluide, qui peut être introduit dans l'espace (24, 25, 24a) par l'intermédiaire du canal (22, 26, 22a), une pression puisse être établie dans ledit espace (24, 25, 24a) pour séparer l'élément rapporté (3, 3a) de la coque (2, 2a), et
dans laquelle le canal (22, 26, 22a) est réalisé dans la coque (2, 2a) et les parois du canal (22, 26, 22a) sont constituées par la coque.

2. Glène artificielle selon la revendication 1,
**caractérisée en ce que** l'espace (24, 24a) s'étend au-dessous du fond (33, 33a) de l'élément rapporté (3, 3a).

3. Glène artificielle selon la revendication 1 ou 2,
**caractérisée en ce que** l'espace s'étend entre le fond (23) de la coque (2) et le fond (33) de l'élément rapporté (3).

4. Glène artificielle selon l'une des revendications 1 à 3,
**caractérisée en ce que** l'espace (24a) est réalisé sous la forme d'une rainure annulaire, prévue dans la coque (2a) et fermée vers le haut par le fond (33a) de l'élément rapporté (3a).

5. Glène artificielle selon l'une des revendications 1 à 4,
**caractérisée en ce que** l'espace (25) est réalisé entre la paroi latérale (21) de la coque (2) et la paroi latérale (31) de l'élément rapporté (3).

6. Glène artificielle selon la revendication 5,
**caractérisée en ce que** l'espace (25) est réalisé sous la forme d'une rainure périphérique dans la paroi latérale (21) de la coque (2).

7. Glène artificielle selon l'une des revendications 1 à 6,
**caractérisée en ce qu'**un ajustement pressé est réalisé entre l'élément rapporté (3, 3a) et la coque (2, 2a).

8. Glène artificielle selon l'une des revendications 1 à 7,
**caractérisée en ce que** l'élément rapporté (3, 3a) est solidement agencé dans la coque (2, 2a) à l'aide d'une liaison conique.

9. Glène artificielle selon la revendication 8,
**caractérisée en ce que**, pour réaliser la liaison conique, la paroi latérale (21) de la coque (2) et la paroi latérale (31) de l'élément rapporté (3) sont pourvues d'une surface conique.

10. Glène artificielle selon l'une des revendications 1 à 9,
**caractérisée en ce que** l'élément rapporté (3a) est solidement agencé dans la coque (2a) à l'aide d'un assemblage à enclenchement.

11. Glène artificielle selon la revendication 10,
**caractérisée en ce que** l'assemblage à enclenchement est agencé à proximité du fond de la coque (2a).

12. Glène artificielle selon la revendication 10 ou 11,
**caractérisée en ce que** l'assemblage à enclenchement est formé par des parties (36a, 26a), qui sont situées sur l'élément rapporté (3a) et sur la coque (2a) et qui viennent en prise mutuelle à contre-dépouille.

13. Glène artificielle selon l'une des revendications 1 à 12,
**caractérisée en ce que** l'élément rapporté (3) est fabriqué en céramique ou en métal.

14. Glène artificielle selon l'une des revendications 1 à 12,
**caractérisée en ce que** l'élément rapporté (3a) est fabriqué en polyéthylène.
